# EUROPÄISCHE PATENTSCHRIFT

(11) **EP 3 624 872 B1**
(45) Veröffentlichungstag und Bekanntmachung des Hinweises auf die Patenterteilung: **19.02.2025**
(21) Anmeldenummer: 18728540.8
(22) Anmeldetag: 16.05.2018
(51) Int. Cl.: A61M 1/28

(54) **PERITONEALDIALYSEGERÄT**
PERITONEAL DIALYSIS MACHINE
APPAREIL DE DIALYSE PÉRITONÉALE

(30) Priorität: 16.05.2017 DE 102017110607
(43) Veröffentlichungstag der Anmeldung: 25.03.2020
(73) Patentinhaber: Fresenius Medical Care Deutschland GmbH, 61352 Bad Homburg (DE)
(72) Erfinder: WOLF, Klaus, 97450 Muedesheim (DE); WABEL, Peter, 61191 Rosbach (DE)
(74) Vertreter: Herrmann, Uwe
(86) Internationale Anmeldenummer: PCT/EP2018/062816
(87) Internationale Veröffentlichungsnummer: WO 2018/210973

(56) Entgegenhaltungen:
- WO-A1-2013/141896
- US-A1- 2009 012 455

## Beschreibung

Die Erfindung betrifft ein Gerät für die Durchführung einer Peritonealdialysebehandlung an einem Patienten.

Die Peritonealdialyse (PD) ist auch unter dem Begriff Bauchfelldialyse bekannt. Es gibt unterschiedliche PD-Verfahren, darunter die mit Peritonealdialysegeräten durchgeführten Verfahren der automatisierten Peritonealdialyse (APO). Bei der APO werden alle oder zumindest einige der Behandlungsschritte automatisiert durchgeführt. Dies können beispielsweise das Ein- oder Ausschalten von Pumpen, das Öffnen oder Schließen von Ventilen etc. sein.

Das Bewirken einer Strömung von Dialyselösung kann gravimetrisch, d.h. schwer- kraftbedingt, und/oder mittels einer oder mehrere Pumpen erfolgen.

Die vorliegende Erfindung ist auf kein besondere Art der PD beschränkt, d.h. umfasst sowohl automatische, nicht automatische, gravimetrische sowie auch mit Pumpen arbeitenden Geräte.

Bei der PD führt das Gerät dem Patienten z.B. automatisiert mittels einer Pumpe oder gravimetrisch in einer Einlaufphase über einen Katheter eine Dialyselösung in die Bauchhöhle.

Die Dialyselösung wird dann während einer Halteperiode in der Bauchhöhle belassen. Dabei können kleinmolekulare Substanzen aus dem Blut über die Kapillargefäße des Bauchfells in die Dialyselösung übertreten, da ein Konzentrationsgefälle herrscht. ferner kann dem Körper auf diesem Wege auch Wasser entzogen werden, sofern die Dialyselösung einen höheren Gehalt an osmotisch aktiven Substanzen aufweist als das Blut. Nach Ende der Halteperiode entfernt das Gerät die mit ausgeschiedenen Substanzen angereicherte und mithin verbrauchte Dialyselösung in einer Ablaufphase über den Katheter wieder aus der Bauchhöhle. Der Flüssigkeitstausch kann gravimetrisch oder aktiv mittels einer Pumpe erfolgen.

Der Zyklus aus Einlaufphase, Halteperiode und Ablaufphase wird bei typischen Verfahrensfühungen mehrmals wiederholt, beispielsweise in der Nacht, während der Patient schläft. Eine neue Einlaufphase wird immer dann gestartet, wenn das Gerät feststellt, dass die Ablaufphase beendet ist, d.h., dass die verbrauchte Dialyseflüssigkeit vollständig oder teilweise aus der Bauchhöhle des Patienten abgelaufen ist.

Derzeit bekannte Peritonealdialysegeräte treffen die Feststellung, ob eine Ablaufphase beendet ist und eine neue Einlaufphase beginnen kann, rein anhand von Kriterien, die sich normalerweise am Ende der Ablaufphase einstellen, also z.B. erreichtes Minimalablaufvolumen und Unterschreitung einer bestimmten Flussrate. Hierbei kann es vorkommen, dass Störungen im Ablauf, die sich beispielsweise durch ein unbeabsichtigtes Knicken des Drainageschlauchs ergeben, nicht erkannt werden und dass die Ablaufphase so vorzeitig abgebrochen wird und die nachfolgende Einlaufphase zu früh startet. Im Interesse einer möglichst effizienten Behandlung ist es aber erstrebenswert, dass in jeder Ablaufphase die verbrauchte Dialyselösung möglichst vollständig aus der Bauchhöhle entfernt wird, um möglichst große Austauschvolumina sicherzustellen. Aus der US2009012455A1 ist z.B. ein Dialysesystem bekannt. WO2013/141896A1 offenbart ein bekanntes Peritonealdialysesystem.

Aufgabe der Erfindung ist es, ein Peritonealdialysegerät bereitzustellen, mit dem der Fortschritt des Ablaufs bzw. das tatsächliche Ende der Ablaufphase zuverlässiger erkannt werden kann.

Vor diesem Hintergrund betrifft die Erfindung ein Peritonealdialysegerät, vorzugsweise für die Durchführung einer automatisierten Peritonealdialysebehandlung mit sich wiederholenden Zyklen, die Zyklen umfassend eine Einlaufphase, eine Halteperiode und eine Ablaufphase für die Dialyseflüssigkeit, wobei das Gerät eine Steuereinheit und eine Messvorrichtung zur Bestimmung der Einlauf- und/oder Abflussrate der Dialyseflüssigkeit von einem Patienten aufweist. Erfindungsgemäß ist vorgesehen, dass die Steuereinheit ausgebildet ist, einen Vergleich zwischen einer von der Messvorrichtung bestimmten zeitlichen Verlaufskurve der Einlauf- und/oder Abflussrate während einer Einlauf- und/oder Ablaufphase mit einer korrespondierenden Referenzkurve vorzunehmen und auf der Grundlage dieses Vergleichs eine Störung im Einlauf und/oder Ablauf der Dialyseflüssigkeit zum bzw. vom Patienten zu erkennen.

Vorzugsweise ist das Gerät derart ausgebildet, dass der Einlauf und der Ablauf automatisiert mittels einer Steuereinheit des Gerätes erfolgen.

Es kann vorgesehen sein, dass eine Referenzkurve für den zeitlichen Verlauf der Abflussrate während der Einlauf- und/oder Ablaufphase in der Steuereinheit hinterlegt ist, mit der eine gemessene Vergleichskurve verglichen wird. Anhand des Vergleichs kann ein atypischer zeitlicher Verlauf der Einlauf- und/oder Abflussrate erkannt werden, der auf eine Störung in der Einlauf- und/oder Ablaufphase hindeutet. Auf dieser Grundlage kann beispielsweise die Erkennungsgenauigkeit verbessert werden, ob wirklich die gesamte bzw. die gewünschte Menge der Dialyseflüssigkeit vom Patienten abgelaufen ist. Ferner können Gegenmaßnahmen im Falle einer Störung eingeleitet werden.

Die vorliegende Erfindung betrifft vorzugsweise aktiv fördernde APO-Geräte, d.h. Peritonealdialysegeräte, bei denen der Einlauf und/oder der Ablauf der Dialyselösung in den/aus dem Patienten mittels einer oder mehrerer Pumpen erfolgt, sowie auch gravimetrisch arbeitenden Peritonealdialysegeräte, bei denen der genannte Einlauf und/oder Ablauf mittels der Schwerkraft und ohne Pumpen erfolgt. Die Steuerung der Fluidströme erfolgt in beiden Fällen vorzugsweise automatisiert, z.B. durch Öffnen und Schließen von Ventilen etc.

Erfindungsgemäß ist vorgesehen, dass die Steuereinheit so ausgebildet ist, dass bei Erkennung einer Störung im Ablauf der Dialyseflüssigkeit vom Patienten eine zeitlich begrenzte Beobachtungsphase gestartet wird, während welcher der die Abflussrate gemessen und auf das Wiedererreichen eines Minimalflusses überprüft wird. Es wird in dieser Ausführungsform also abgewartet, ob sich wieder ein Minimalfluss einstellt, beispielsweise weil sich ein Knick im Katheter bzw. im Schlauch durch Bewegung des Patienten von selbst wieder löst. Wird während der Beobachtungsphase, die beispielsweise zwischen 1 und 5 Minuten dauern kann, der angestrebte Minimalfluss nicht wieder erreicht, können weitere Maßnahmen vorgesehen sein. Der Minimalfluss kann beispielsweise demjenigen Fluss entsprechen, der in der Referenzkurve zu einem bestimmten Zeitpunkt der Ablaufphase vorgesehen wäre, gegebenenfalls korrigiert um einen Toleranzabschlag.

In einer Ausführungsform ist vorgesehen, dass die Steuereinheit so ausgebildet ist, dass bei Erkennung einer Störung im Ablauf der Dialyseflüssigkeit vom Patienten ein Volumen in Richtung des Patienten gefördert wird. Anhand dieser Maßnahme kann die Qualität der Leitung geprüft werden. Die Förderung kann beispielsweise aktiv mittels einer Fluidpumpe erfolgen. Die Maßnahme kann gegebenenfalls nach Ablauf einer Beobachtungsphase ergriffen werden, sofern eine solche vorgesehen ist.

in einer Ausführungsform ist vorgesehen, dass die Steuereinheit so ausgebildet ist, dass bei Erkennung einer Störung im Einlauf und/oder Ablauf der Dialyseflüssigkeit zum bzw. vom Patienten ein Signal ausgegeben wird.

Zu diesem Zweck kann vorgesehen sein, dass das Gerät eine Signaleinheit oder eine Schnittstelle zur Kommunikation mit einer externen Signaleinheit aufweist. Die Signalausgabe kann einen Versuch darstellen, die Situation zu verbessern. Beispielsweise kann das Signal dazu dienen, den Patienten aufzuwecken bzw. zu stimulieren oder darauf aufmerksam zu machen, dass er sich bewegen oder den Katheter aktiv wieder in Betrieb setzen soll. Geeignete Signale umfassen beispielsweise optische Signale, Tonsignale oder Vibrationssignale. Die Maßnahme kann gegebenenfalls nach Ablauf einer Beobachtungsphase und/oder nach Förderung eines Volumens in Richtung des Patienten ergriffen werden, sofern derartige Maßnahmen vorgesehen sind.

in einer Ausführungsform ist vorgesehen, dass die Steuereinheit so ausgebildet ist, dass auf der Grundlage des Vergleichs eine Optimierung der Gerätekonfiguration vorgenommen wird. Beispielsweise können Kriterien, wann eine Ablaufphase als beendet anzusehen ist, entsprechend der Referenzkurve und der gemessenen Kurve auf einen Patienten angepasst werden.

In einer Ausführungsform ist vorgesehen, dass es sich bei der Referenzkurve um eine patientenunabhängige Funktion handelt, die in der Steuereinheit hinterlegt ist. Die Referenzkurve kann beispielsweise schematisch anhand von Verschreibungsparametern, wie etwa der Menge der verabreichten Flüssigkeit, festgelegt werden.

In einer Ausführungsform ist vorgesehen, dass es sich bei der Referenzkurve um eine patientenindividuelle Funktion handelt. Beispielsweise kann vorgesehen sein, dass eine Referenzkurve in einem Einstellzyklus vor Behandlungsbeginn aufgezeichnet wird. Ferner kann beispielsweise vorgesehen sein, dass die Referenzkurve aus einer Mehrzahl an gemessenen Kurven z.B. durch Mittelwertbildung ermittelt wird. Die Referenzkurve kann durch eine Überlagerung von Kurven gebildet werden, die in einer bestimmten Anzahl an vorangegangenen Zyklen bzw. Behandlungen gemessen wurden, es kann sich also um einen patientenindividuellen Mittelwert handeln.

In einer Ausführungsform ist vorgesehen, dass es sich bei dem Gerät um ein gravimetrisch arbeitendes Gerät handelt. Beispielsweise können ein oder mehrere Ventile vorgesehen und die Steuereinheit so ausgebildet sein, dass das oder ein Ventil nach der Haltephase und vor Beginn der Ablaufphase geöffnet wird, um einen gravimetrischen Ablauf von Dialyseflüssigkeit vom Patienten zu ermöglichen, und/oder dass das oder ein Ventil nach der Ablaufphase und vor Beginn der Einlaufphase geöffnet wird, um einen gravimetrischen Einlauf von Dialyseflüssigkeit zum Patienten zu ermöglichen.

In einer Ausführungsform ist vorgesehen, dass es sich bei dem Gerät um ein aktiv förderndes Gerät handelt. Beispielsweise können ein oder mehrere Pumpen vorgesehen und die Steuereinheit so ausgebildet sein, dem Patienten während der Ablaufphase Dialyseflüssigkeit anhand der oder einer Pumpe zu entziehen und/oder dem Patienten während der Einlaufphase Dialyseflüssigkeit anhand der oder einer Pumpe zuzuführen.

Ferner umfasst die Erfindung ein Verfahren zur Durchführung einer Peritonealdialyse unter Verwendung eines erfindungsgemäßen Peritonealdialysegeräts, wobei ein Vergleich zwischen einer gemessenen zeitlichen Verlaufskurve der Einlauf- und/oder Abflussrate während einer Einlauf- und/oder Ablaufphase mit einer korrespondierenden Referenzkurve vorgenommen wird, und wobei auf der Grundlage dieses Vergleichs eine Störung im Einlauf und/oder Ablauf der Dialyseflüssigkeit zum bzw. vom Patienten erkannt wird. Vorteilhafte Ausgestaltungen des Verfahrens ergeben sich aus der obigen Beschreibung der Ausbildung der Steuereinheit im erfindungsgemäßen Peritonealdialysesystem.

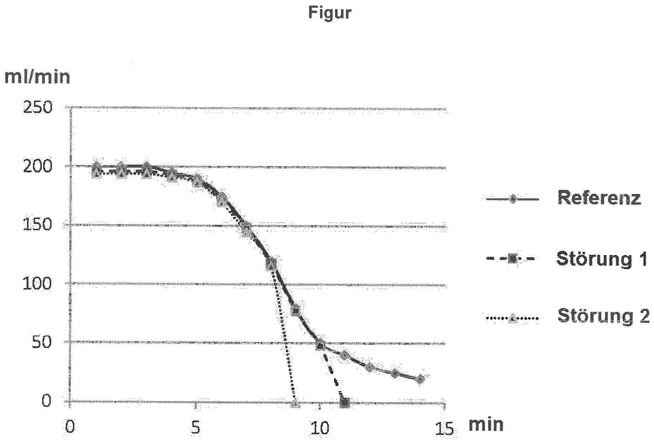

Weitere Einzelheiten und Vorteile der Erfindung ergeben sich aus dem nachfolgend anhand der Figur erklärten Ausführungsbeispiel. Die einzige Figur zeigt mögliche zeitliche Verläufe der Abflussrate (Volumen/Zeit) in der Bauchhöhle während der Ablaufphase bei gravimetrischem, d.h., passivem Ablauf.

Gemäß dem Ausführungsbeispiel ist ein Peritonealdialysegerät vorgesehen, welches für die Durchführung einer automatisierten Peritonealdialysebehandlung mit sich wiederholenden Zyklen umfassend eine Einlaufphase, eine Halteperiode und eine Ablaufphase für die Dialyseflüssigkeit bestimmt ist. Das Gerät umfasst eine Steuereinheit und eine Messvorrichtung zur Bestimmung der Einlauf- und/oder Abflussrate der Dialyseflüssigkeit von einem Patienten. Es umfasst ferner eine Dialysatpumpe zum Fördern von Dialyseflüssigkeit an den Patienten, sowie ein Ablaufventil, das von der Steuereinheit nach der Haltephase und vor Beginn der Ablaufphase geöffnet wird, um einen gravimetrischen Ablauf von Dialyseflüssigkeit vom Patienten zu ermöglichen.

Am Speicher z.B. der Steuereinheit ist eine Referenzkurve für den zeitlichen Verlauf der Auslaufphase hinterlegt, die patientenindividuell ist und durch eine Überlagerung von Kurven gebildet wurde, die im Rahmen von Abläufen in einer bestimmten Anzahl an vorangegangenen Zyklen gemessen wurden. Ferner ist auf der Steuereinheit ein Algorithmus hinterlegt, anhand dessen ein Vergleich zwischen einer von der Messvorrichtung bestimmten zeitlichen Verlaufskurve der Abflussrate während einer weiteren Ablaufphase mit der Referenzkurve vorgenommen werden kann. Der Algorithmus erlaubt es ferner, auf der Grundlage dieses Vergleichs eine Störung im Ablauf der Dialyseflüssigkeit vom Patienten zu erkennen.

Anstatt eines Speichers, der sich physisch im Gerät befindet, kann sich der Speicher auch auf beweglichen Gegenstand, wie z.B. auf einer Patientenkarte befinden oder ein sonstiger Datenspeicher sein, der keinen Bestandteil des Gerätes bildet.

Sofern eine Störung erkannt wird, ist die Steuereinheit ausgebildet, eine Reihe von Maßnahmen zu ergreifen.

Zunächst wird eine bestimmte, beispielsweise dreiminütige, Beobachtungsphase gestartet, in welcher die Abflussrate fortlaufend gemessen und auf das Wiedererreichen eines Minimalflusses überprüft wird. Der Minimalfluss entspricht demjeni- gen Fluss, vor Auftreten der Störung vorhanden sein sollte, abzüglich eines bestimmten, beispielsweise 30%igen, Toleranzintervalls.

Sofern im Rahmen der Beobachtungsphase der Minimalfluss nicht wieder erreicht wird, startet die Steuereinheit die Dialysatpumpe, um ein bestimmtes, kleineres Volumen von beispielsweise 100 ml in Richtung des Patienten zu fördern. Anhand dieser Maßnahme kann die Qualität der Leitung geprüft werden.

Bei den vorgenannten Zahlenwerten handelt es sich um Beispiele, die die Erfindung nicht beschränken.

Alternativ oder zusätzlich kann ein Warnsignal, vorzugsweise eine akustische Information ausgegeben werden.

Ergibt sich im Rahmen dieses Befülltests eine Störung, die beispielsweise auf einen Knick im Katheter hindeuten könnte, so gibt die Steuereinheit an einer im Gerät verbauten Signaieinheit ein akustisches Signal aus. Es soll geeignet sein, den Patienten zu wecken und darauf aufmerksam zu machen, seine Position zu verändern.

Nach diesem Schritt erfolgt eine Reevaluierung in Form einer erneuten Beobachtungsphase und eines erneuten Befülltests. Auch die Signalisierung kann in derselben oder in intensiverer Form wiederholt werden, dass der Patient dann ggf. tatsächlich aufsteht, um die Funktion der Leitungen zu überprüfen.

Die Kurve "Referenz" der Figur zeigt einen zeitlichen Verlauf der Abflussrate in einem Gerät gemäß Ausführungsbeispiel im Normalfall. Die Abflussrate nimmt aufgrund der zunehmehenden Entleerung des Patienten aufgrund des geringer werdenden hydrostatischen Drucks ab. Diese Zeitspanne kann beispielsweise 5 min betragen.

Die Kurve "Störung 1" der Figur zeigt einen zeitlichen Verlauf der Abflussrate für den Fall, dass der Abfluss kurz vor dem Ende der Ablaufphase gestört wird. Dieser Fall kann beispielsweise eintreten, wenn der Ablaufkatheter durchhängt oder anderweitig verstopft oder geklemmt/genickt ist, sodass der weitere Ablauf bei Unterschreitung eines bestimmten hydrostatischen Drucks nicht mehr weiterläuft.

Die Kurve "Störung 2" der Figur zeigt schließlich einen zeitlichen Verlauf der Abflussrate für den Fall, dass der Abfluss noch bereits etwas früher gestört wird. Dieser Fall kann beispielsweise eintreten, wenn der Ablaufkatheter durch eine Bewegung des Patienten im Schlaf geknickt oder anderweitig blockiert wird, wie z.B. verstopft ist, sodass der weitere Ablauf bei Unterschreitung eines bestimmten hydrostatischen Drucks nicht mehr weiterläuft.

Vorteile der erfindungsgemäßen Lösung umfassen beispielsweise eine Vermeidung eines frühzeitigen Umschaltens von einer Auslaufphase in die Einlaufphase des nachfolgenden Zyklus, sowie insgesamt eine Verbesserung des Auslaufverhaltens und mithin eine Optimierung der Therapie. Die Zahl der unberechtigt bzw. ungewollt beendeten Auslaufphasen kann reduziert werden. Es bestehen auch weniger Möglichkeiten, dass der Anwender das Geräteverhalten negativ beeinflusst. Der Patient bekommt ein verbessertes Feedback beispielsweise mit Blick auf seine Schlafposition, die Betthöhe oder den Aufsteliort des Gerätes.

## Patentansprüche

1. Peritonealdialysegerät für die Durchführung einer Peritonealdialysebehandlung mit sich wiederholenden Zyklen, die Zyklen umfassend eine Einlaufphase, eine Halteperiode und eine Ablaufphase für die Dialyseflüssigkeit, wobei das Gerät eine Steuereinheit und eine Messvorrichtung zur Bestimmung der Einlauf- und/oder Abflussrate der Dialyseflüssigkeit zu/von einem Patienten aufweist,
**dadurch gekennzeichnet,**
**dass** die Steuereinheit ausgebildet ist, einen Vergleich zwischen einer von der Messvorrichtung bestimmten zeitlichen Verlaufskurve der Einlauf- und/oder Abflussrate während einer Einlauf- und/oder Ablaufphase mit einer korrespondierenden Referenzkurve vorzunehmen und auf der Grundlage dieses Vergleichs eine Störung im Einlauf und/oder Ablauf der Dialyseflüssigkeit zum bzw. vom Patienten zu erkennen, und
**dass** die Steuereinheit so ausgebildet ist, dass bei Erkennung einer Störung im Ablauf der Dialyseflüssigkeit vom Patienten und/oder bei Erkennung einer Störung im Einlauf der Dialyseflüssigkeit zum Patienten eine zeitlich begrenzte Beobachtungsphase gestartet wird, während welcher der die Abflussrate bzw. Einflussrate gemessen und auf das Wiedererreichen eines Flusses oder eines Minimalflusses überprüft wird.

2. Peritonealdialysegerät nach einem der vorhergehenden Ansprüche, **dadurch gekennzeichnet, dass** die Steuereinheit so ausgebildet ist, dass bei Erkennung einer Störung im Ablauf der Dialyseflüssigkeit vom Patienten ein Volumen in Richtung des Patienten gefördert wird.

3. Peritonealdialysegerät nach einem der vorhergehenden Ansprüche, **dadurch gekennzeichnet, dass** die Steuereinheit so ausgebildet ist, dass bei Erkennung einer Störung im Einlauf und/oder Ablauf der Dialyseflüssigkeit zum bzw. vom Patienten ein Signal ausgegeben wird.

4. Peritonealdialysegerät nach Anspruch 3, **dadurch gekennzeichnet, dass** es sich bei dem Signal um ein optisches Signal, ein akustisches Signale oder ein Vibrationssignal handelt.

5. Peritonealdialysegerät nach einem der vorhergehenden Ansprüche, **dadurch gekennzeichnet, dass** die Steuereinheit so ausgebildet ist, dass auf der Grundlage des Vergleichs eine Optimierung der Gerätekonfiguration vorgenommen wird.

6. Peritonealdialysegerät nach einem der vorhergehenden Ansprüche, **dadurch gekennzeichnet, dass** es sich bei der Referenzkurve um eine patientenunabhängige Funktion handelt, die in der Steuereinheit hinterlegt ist.

7. Peritonealdialysegerät nach einem der Ansprüche 1 bis 5, **dadurch gekennzeichnet, dass** es sich bei der Referenzkurve um eine patientenindividuelle Funktion handelt.

8. Peritonealdialysegerät nach einem der vorhergehenden Ansprüche, **dadurch gekennzeichnet, dass** es sich bei dem Gerät um ein gravimetrisch arbeitendes Gerät handelt.

9. Peritonealdialysegerät nach einem der Ansprüche 1 bis 7, **dadurch gekennzeichnet, dass** es sich bei dem Gerät um ein aktiv förderndes Gerät handelt.

## Claims

1. A peritoneal dialysis machine for the carrying out of a peritoneal dialysis treatment having recurring cycles, the cycles comprising an inflow phase, a dwell period and a drainage phase for the dialysis fluid, wherein the machine has a control unit and a measurement apparatus for determining the inflow and/or drainage rate of the dialysis fluid to and from a patient,
**characterized in that**
the control unit is configured to carry out a comparison between a time progression curve of the inflow rate and/or of the drainage rate determined by the measurement apparatus during an inflow phase and/or a drainage phase and a corresponding reference curve and to recognize on the basis of this comparison a disturbance in the inflow and/or drainage of the dialysis fluid to and from the patient; and
**in that** the control unit is configured such that, on a recognition of a disturbance in the drainage of the dialysis fluid from the patient and/or on a recognition of a disturbance in the inflow of dialysis fluid to the patient, a time-limited observation phase is started during which the drainage rate or the inflow rate is measured and a check is made with respect to the re-reaching of a flow or of a minimal flow.

2. A peritoneal dialysis machine in accordance with one of the preceding claims, **characterized in that** the control unit is configured such that, on a recognition of a disturbance in the drainage of the dialysis fluid from the patient, a volume is conveyed in the direction of the patient.

3. A peritoneal dialysis machine in accordance with one of the preceding claims, **characterized in that** the control unit is configured such that a signal is output on a recognition of a disturbance in the inflow and/or drainage of the dialysis fluid to or from the patient.

4. A peritoneal dialysis machine in accordance with claim 3, **characterized in that** the signal is a visual signal, an acoustic signal or a vibration signal.

5. A peritoneal dialysis machine in accordance with one of the preceding claims, **characterized in that** the control unit is configured such that an optimization of the machine configuration is carried out on the basis of the comparison.

6. A peritoneal dialysis machine in accordance with one of the preceding claims, **characterized in that** the reference curve is a patient-independent function that is stored in the control unit.

7. A peritoneal dialysis machine in accordance with one of the claims 1 to 5, **characterized in that** the reference curve is a patient-individual function.

8. A peritoneal dialysis machine in accordance with one of the preceding claims, **characterized in that** the machine is a gravimetrically working machine.

9. A peritoneal dialysis machine in accordance with one of the claims 1 to 7, **characterized in that** the machine is an actively conveying machine.

## Revendications

1. Appareil de dialyse péritonéale pour effectuer un traitement de dialyse péritonéale avec des cycles répétitifs, les cycles comprenant une phase d'entrée, une période de rétention et une phase de sortie du liquide de dialyse, l'appareil présentant une unité de commande et un dispositif de mesure pour déterminer le débit d'entrée et/ou d'écoulement du liquide de dialyse vers/depuis un patient,
**caractérisé en ce que**
l'unité de commande est conçue pour effectuer une comparaison entre une courbe de variation dans le temps déterminée par le dispositif de mesure du débit d'entrée et/ou d'écoulement pendant une phase d'entrée et/ou de sortie et une courbe de référence correspondante et, sur la base de cette comparaison, détecter un dysfonctionnement dans l'entrée et/ou la sortie du liquide de dialyse vers ou depuis le patient, et
**en ce que** l'unité de commande est conçue de telle manière que, lors de la détection d'un dysfonctionnement dans la sortie du liquide de dialyse depuis le patient et/ou lors de la détection d'un dysfonctionnement dans l'entrée du liquide de dialyse vers le patient, une phase d'observation limitée dans le temps est démarrée, pendant laquelle le débit d'écoulement ou le débit d'entrée est mesuré et une vérification à lieu pour savoir s'il atteint de nouveau un flux ou un flux minimal.

2. Appareil de dialyse péritonéale selon l'une des revendications précédentes, **caractérisé en ce que** l'unité de commande est conçue de telle manière que, lors de la détection d'un dysfonctionnement dans la sortie du liquide de dialyse depuis le patient, un volume est refoulé en direction du patient.

3. Appareil de dialyse péritonéale selon l'une des revendications précédentes, **caractérisé en ce que** l'unité de commande est conçue de telle manière que, lors de la détection d'un dysfonctionnement dans l'entrée et/ou la sortie du liquide de dialyse vers ou depuis le patient, un signal est émis.

4. Appareil de dialyse péritonéale selon la revendication 3, **caractérisé en ce que** le signal est un signal optique, un signal sonore ou un signal vibratoire.

5. Appareil de dialyse péritonéale selon l'une des revendications précédentes, **caractérisé en ce que** l'unité de commande est conçue de telle manière que, sur la base de la comparaison, une optimisation de la configuration de l'appareil est effectuée.

6. Appareil de dialyse péritonéale selon l'une des revendications précédentes, **caractérisé en ce que** la courbe de référence est une fonction indépendante du patient qui est enregistrée dans l'unité de commande.

7. Appareil de dialyse péritonéale selon l'une des revendications 1 à 5, **caractérisé en ce que** la courbe de référence est une fonction propre au patient.

8. Appareil de dialyse péritonéale selon l'une des revendications précédentes, **caractérisé en ce que** l'appareil est un appareil fonctionnant par gravimétrie.

9. Appareil de dialyse péritonéale selon l'une des revendications 1 à 7, **caractérisé en ce que** l'appareil est un appareil à refoulement actif.
